# EUROPEAN PATENT APPLICATION

(11) **EP 4 752 889 A1**
(43) Date of publication of application: **03.06.2026**
(21) Application number: 25215614.6
(22) Date of filing: 13.11.2025
(51) Int. Cl.: G16C 20/70, G06Q 10/10, G16C 20/90

(54) **ANALYSIS SYSTEM, ANALYSIS METHOD, AND ANALYSIS PROGRAM**

(30) Priority: 02.12.2024 JP 2024209485
(71) Applicant: Toyota Jidosha Kabushiki Kaisha, Toyota-shi, Aichi-ken 471-8571 (JP)
(72) Inventor: SAEKI, Kazuho, Toyota-shi, 471-8571 (JP)
(74) Representative: J A Kemp LLP

(57) **Abstract**

An analysis system, an analysis method, and an analysis program capable of assisting analysis that utilizes knowledge possessed by an experimenter are provided. An analysis system (1) includes a text acquisition unit (114), an extraction unit (115), and a difference evaluation unit (116). The text acquisition unit (114) acquires text data corresponding to each of a plurality of samples. The extraction unit (115) extracts extraction information about the samples from the text data. The difference evaluation unit (116) evaluates a difference between a plurality of pieces of the extraction information.

## Description

### BACKGROUND

The present disclosure relates to an analysis system, an analysis method, and an analysis program.

Patent Literature 1 discloses a data analysis system that analyzes measurement data obtained by analyzing materials. The data analysis system disclosed in Patent Literature 1 receives measurement data through a communication apparatus, processes the measurement data using a trained machine learning model, and outputs a result of the analysis.

[Patent Literature 1] Japanese Unexamined Patent Application Publication No. 2021-092467

### SUMMARY

It should be noted that experimenters and developers obtain various kinds of knowledge in experiments. Such knowledge may include important information in analysis.

However, it is difficult to incorporate the above knowledge into analysis. Therefore, in the analysis system according to the related art, the above knowledge cannot be sufficiently utilized.

That is, in the analysis system according to the related art, there is a problem that knowledge obtained by an experimenter in an experiment cannot be sufficiently utilized. Patent Literature 1 does not disclose any technology that can solve such a problem.

The present disclosure has been made to solve such a problem, and an object thereof is to provide a display system, a display method, and a display program that are capable of assisting analysis that utilizes knowledge possessed by an experimenter.

An analysis system according to the present disclosure includes a text acquisition unit, an extraction unit, and a difference evaluation unit. The text acquisition unit acquires text data corresponding to each of a plurality of samples. The extraction unit extracts extraction information about the samples from the text data. The difference evaluation unit evaluates a difference between a plurality of pieces of the extraction information.

According to the above configuration, the analysis system can, for example, extract a difference between samples whose importance is not recognized by a user. As a result, an analysis system 1 according to the embodiment can assist analysis that utilizes knowledge possessed by an experimenter.

In the analysis system according to the present disclosure, the extraction information may include at least one of information about experimental conditions of the samples and information about physical properties of the samples.

In the analysis system according to the present disclosure, the extraction unit may extract an item related to at least one of the information about the experimental conditions of the samples and the information about the physical properties of the samples.

The analysis system according to the present disclosure may further include a notification unit configured to notify a user about an item regarding which the difference evaluation unit has evaluated that there is a difference between the plurality of pieces of the extraction information.

The analysis system according to the present disclosure may further include a group generation unit configured to generate a first sample group and a second sample group by receiving selection of the sample from a user. Further, the difference evaluation unit may evaluate a difference between the extraction information corresponding to the first sample group and the extraction information corresponding to the second sample group.

The analysis system according to the present disclosure may further include an analysis subject data acquisition unit configured to acquire analysis subject data including at least one of measurement data obtained by measuring samples and numerical data about the samples, and a display control unit configured to display a plurality of pieces of the analysis subject data. Further, the group generation unit may receive the selection of the sample by making a user select the displayed analysis subject data.

The analysis system according to the present disclosure may further include an analysis subject data acquisition unit and a group generation unit. The analysis subject data acquisition unit acquires analysis subject data including at least one of measurement data obtained by measuring samples and numerical data about the samples. The group generation unit performs clustering processing on at least one of the analysis subject data and a result of analysis of the analysis subject data, and generates a first sample group and a second sample group based on a result of the clustering processing. Further, the difference evaluation unit evaluates a difference between the extraction information corresponding to the first sample group and the extraction information corresponding to the second sample group.

An analysis method according to the present disclosure performs the following steps.

Text data corresponding to each of a plurality of samples is acquired. Extraction information about the samples is extracted from the text data. A difference between a plurality of pieces of the extraction information is evaluated.

An analysis program according to the present disclosure causes a computer to perform the following operations.

Text data corresponding to each of a plurality of samples is acquired.

Extraction information about the samples is extracted from the text data.

A difference between a plurality of pieces of the extraction information is evaluated.

By the present disclosure, it is possible to provide an analysis system, an analysis method, and an analysis program that are capable of assisting analysis that utilizes knowledge possessed by an experimenter.

The above and other objects, features and advantages of the present disclosure will become more fully understood from the detailed description given hereinbelow and the accompanying drawings.

### BRIEF DESCRIPTION OF DRAWINGS

Fig. 1 is a block diagram showing a configuration of an analysis system according to a first embodiment;
Fig. 2 is a block diagram showing a configuration of a server according to the first embodiment;
Fig. 3 is a block diagram showing the configuration of the server according to the first embodiment;
Fig. 4 is a schematic screen diagram for explaining the configuration of the server according to the first embodiment; and
Fig. 5 is a flowchart showing operations performed by the analysis system according to the first embodiment.

### DESCRIPTION OF EMBODIMENTS

### <First Embodiment>

### (Configuration of Analysis system)

A first embodiment according to the present disclosure will be described hereinafter in detail with reference to the drawings. Firstly, a configuration of an analysis system according to this embodiment will be described in detail.

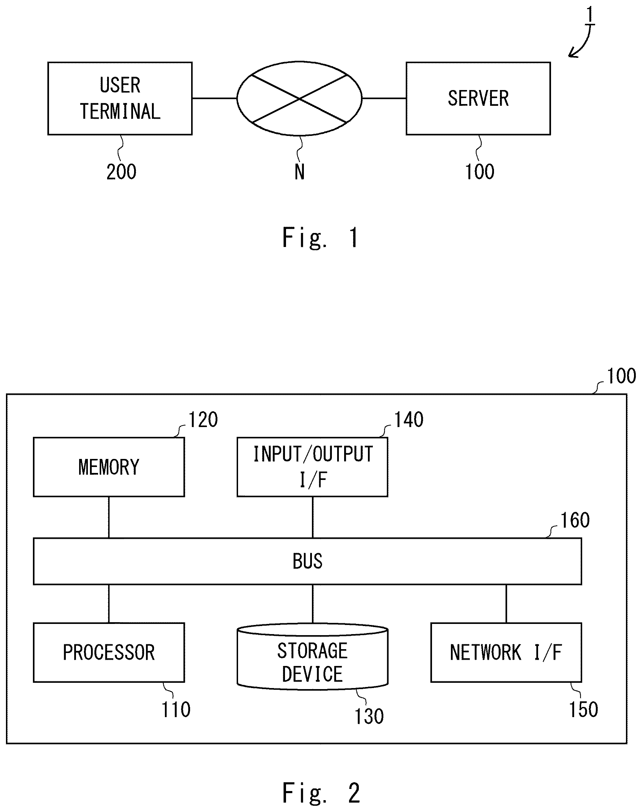

Fig. 1 is a block diagram showing a configuration of an analysis system according to the first embodiment. As shown in Fig. 1, a server 100 and a user terminal 200 are connected to an analysis system 1 according to this embodiment through a network N such as the Internet.

The analysis system 1 according to this embodiment is typically provided as a part of a data cloud type service used in material development or research and development, and is used as a system for promoting research and development using so-called Materials Informatics (MI) or data science.

The analysis system 1 stores various types of data about experimental samples. Further, the analysis system 1 analyzes the stored data based on an instruction from a user.

Note that various types of data about experimental samples described herein include analysis subject data (i.e., data to be analyzed) and text data described later.

In the analysis system 1, the user terminal 200 transmits the various types of data about the experimental samples to the server 100, and the server 100 analyzes the received data. Then the server 100 transmits results of the analysis to the user terminal 200, and the user terminal 200 displays the received results of the analysis.

The user terminal 200 according to this embodiment is a terminal operated by a user, and is typically a computer apparatus including a display apparatus.

The user terminal 200 transmits the various types of data about the experimental samples to the server 100 through the network N. Then the user terminal 200 receives the results of the analysis of the various types of data about the experimental samples from the server 100 through the network N.

The server 100 according to this embodiment receives the various types of data about the experimental samples from the user terminal 200 through the network N, and analyzes the received data. Then the server 100 transmits results of the analysis to the user terminal 200 through the network N.

Fig. 2 is a block diagram showing a hardware configuration of the server according to the first embodiment.

As shown in Fig. 2, the server 100 includes a processor 110, a memory 120, a storage device 130, an input/output interface 140, a network interface 150, and an internal bus 160.

The internal bus 160 is a data transmission path through which the processor 110, the memory 120, the storage device 130, the input/output interface 140, and the network interface 150 transmit and receive data to and from each other. However, the method for connecting the processor 110 and the like to each other is not limited to the bus connection.

The memory 120 is a main storage device implemented by using a Random Access Memory (RAM) or the like. Further, the storage device 130 is an auxiliary storage device implemented by using a hard disk, a Solid State Drive (SSD), a memory card, a Read Only Memory (ROM), or the like. The storage device 130 stores a program(s) for implementing desired functions.

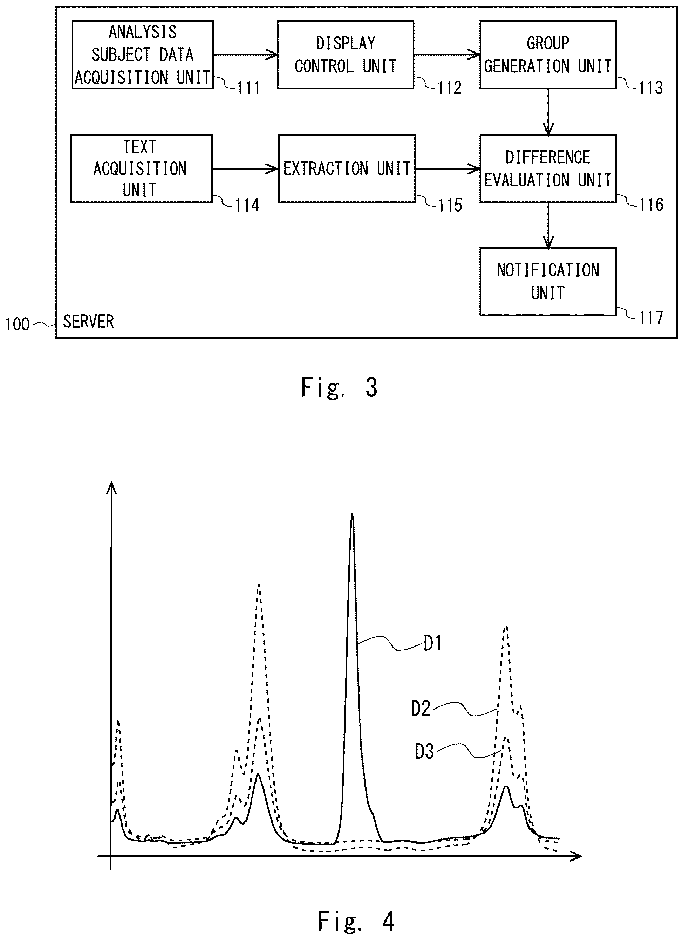

The processor 110 may be a Central Processing Unit (CPU), a Graphics Processing Unit (GPU), or a field-programmable gate array (FPGA). The processor 110 performs the functions of the respective functional blocks shown in Fig. 3 described later by loading the program(s) stored in the storage device 130 into the memory 120 and executing the loaded program(s).

The input/output interface 140 is an interface for connecting the server 100 to input/output devices. For example, an input device such as a keyboard and/or an output device such as a display apparatus may be connected to the input/output interface 140.

The network interface 150 is an interface for connecting the server 100 to the network.

The program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

Fig. 3 is a block diagram showing functions of the server according to the first embodiment. As shown in Fig. 3, the server 100 according to this embodiment includes, as its functional blocks, an analysis subject data acquisition unit 111, a display control unit 112, a group generation unit 113, a text acquisition unit 114, an extraction unit 115, a difference evaluation unit 116, and a notification unit 117.

The analysis subject data acquisition unit 111 acquires analysis subject data. More specifically, the analysis subject data acquisition unit 111 according to this embodiment acquires a plurality of pieces of analysis subject data from the user terminal 200 through the network N. The analysis subject data acquisition unit 111 outputs the acquired analysis subject data to the display control unit 112.

Further, the analysis subject data acquisition unit 111 may store the acquired analysis subject data in the storage device 130 each time it acquires analysis subject data. Further, when analysis described later is performed, the analysis subject data acquisition unit 111 may read the analysis subject data from the storage device 130 and output it to the display control unit 112.

Further, in this case, the analysis subject data acquisition unit 111 does not need to read all pieces of the analysis subject data stored in the storage device 130, and may instead read only the analysis subject data designated by a user and output it to the display control unit 112.

That is, the analysis subject data acquisition unit 111 may create a database in which the analysis subject data is stored. Further, the analysis subject data acquisition unit 111 according to this embodiment may be configured so that a user can select analysis subject data from the created database as appropriate.

Note that analysis subject data acquired by the analysis subject data acquisition unit 111 according to this embodiment is at least one of measurement data obtained by measuring samples and numerical data about the samples.

In other words, the analysis subject data acquisition unit 111 according to this embodiment acquires analysis subject data which is at least one of measurement data obtained by measuring samples and numerical data about the samples.

Note that measurement data obtained by measuring samples may be raw data output from a measuring device which has measured the samples, or data which has been subjected to analysis processing. Measurement data obtained by measuring samples may be any data recorded in computer-readable form.

Examples of measurement data obtained by measuring samples include spectrum data, waveform data, graph data, two-dimensional image data, and three-dimensional image data.

Examples of spectrum data include spectrum data measured by nuclear magnetic resonance spectroscopy (NMR), infrared spectroscopy (IR), ultravioletvisible spectroscopy (UV-vis), X-ray absorption spectroscopy (XAS), Raman spectroscopy, X-ray diffraction (XRD), small-angle X-ray scattering (SAXS), mass spectrometry (MS), or the like.

Further, examples of two-dimensional image data include image data captured by using an optical microscope, a scanning electron microscope (SEM), a transmission electron microscope (TEM), a computed tomography (CT), or the like.

Further, examples of three-dimensional image data include photographic data which is created by stacking tomograms taken by computed tomography (CT), model data created by Computer-Aided design (CAD), or the like.

Further, examples of waveform data include time-series data and displacement data. Examples of time-series data include acoustic data and vibration data, and any data whose numerical value changes with time can be used as analysis subject data. Examples of displacement data include a surface height and a surface profile of a sample, and any data whose numerical value changes as coordinates and other parameters change can be used.

Further, examples of other data include a cyclic voltammogram, a chart graph of gas chromatography (GC), and coordinate data such as a Crystallographic Information (CIF) file.

Numerical data about a sample may be, for example, numerical data defining an experimental condition of a sample or numerical data indicating the composition of a sample.

For example, when a sample is a composition, numerical data about the sample may be the content of each component contained in the composition. Further, when a sample is a product, numerical data about the sample may be, for example, numerical values related to reaction conditions such as a reaction temperature, a reaction time, a weight of a substrate used for reaction, and a reaction scale.

Further, analysis subject data may be data corresponding to both measurement data obtained by measuring samples and numerical data about the samples. Examples of data corresponding to both measurement data obtained by measuring samples and numerical data about the samples include physical property values of the samples.

Examples of physical property values of the samples include a mechanical property value typified by strength, hardness, toughness, abrasion resistance, etc., a physical property value typified by density, conductivity, a magnetic property, thermal conductivity, a thermal expansion rate, etc., and a chemical property value typified by corrosion resistance etc.

Further, analysis subject data is not limited to data related to only a sample, and may include, for example, a performance value of a product and a module created using a sample.

Examples of a performance value of a product and a module created using a sample may include a photoelectric conversion efficiency of a solar cell including a photoelectric conversion layer using a sample as a material and a numerical value obtained by evaluating the durability of a vehicle manufactured using a sample as a body material.

That is, analysis subject data acquired by the analysis subject data acquisition unit 111 may be data directly or indirectly defining the composition of a sample to be analyzed, or data directly or indirectly evaluating the performance of a sample to be analyzed.

Note that analysis subject data according to this embodiment is data in which information about experiment contents are recorded in a form other than text.

The display control unit 112 displays a plurality of pieces of analysis subject data. More specifically, the display control unit 112 according to this embodiment displays a plurality of pieces of analysis subject data on a display apparatus of the user terminal 200.

The display control unit 112 displays pieces of analysis subject data so that a user can select them.

For example, when the analysis subject data is spectrum data, waveform data, or the like, the display control unit 112 may display a plurality of pieces of analysis subject data in a superimposed manner. Further, the display control unit 112 may display pieces of analysis subject data so that a user can select each of the pieces of data displayed in a superimposed manner by, for example, clicking.

Fig. 4 is a schematic screen diagram for explaining the configuration of the server according to the first embodiment. More specifically, Fig. 4 is a schematic screen diagram showing an example of a screen to be displayed on the user terminal 200 by the display control unit 112 according to this embodiment.

In Fig. 4, the display control unit 112 displays three pieces of spectrum data D1, D2, and D3 in a superimposed manner. Note that a user checks the screen displayed on the user terminal 200 and selects analysis subject data.

For example, it is assumed that a user who has checked the screen as shown in Fig. 4 pays attention to the fact that the spectrum data D1 and pieces of the spectrum data D2 and D3 have different features.

In this case, the user selects spectrum data so that the group generation unit 113 described later groups samples corresponding to the spectrum data D1 and samples corresponding to the spectrum data D2 or D3 into different groups.

The analysis system according to this embodiment can search for a difference between samples belonging to different groups from text data associated with the samples, the details of which will be described later.

Note that the difference to be searched for by the analysis system 1 is a difference which may not be regarded as being important by a user or may not be recognized by a user. The analysis system 1 can search for such a difference and present the difference to a user, to thereby make the user recognize the importance of the difference. As a result, the analysis system 1 according to this embodiment can assist analysis that utilizes knowledge possessed by an experimenter.

The group generation unit 113 generates first and second sample groups by receiving selection of a sample from a user. The group generation unit 113 outputs identification information of samples belonging to each group to the difference evaluation unit 116.

More specifically, the group generation unit 113 according to this embodiment receives the selection of the sample by making a user select analysis subject data displayed by the display control unit 112. The group generation unit 113 may, for example, group the samples corresponding to the analysis subject data selected by a user among the samples displayed by the display control unit 112 into the first sample group. Then the group generation unit 113 may group the samples corresponding to the analysis subject data which are not selected by a user into the second sample group.

Note that, although the group generation unit 113 according to this embodiment groups a plurality of samples into two groups, the configuration of the group generation unit according to the present disclosure is not limited thereto.

For example, the group generation unit according to the present disclosure may group a plurality of samples into three or more groups.

Although the group generation unit 113 according to this embodiment groups samples based on selection made by a user, the group generation unit according to the present disclosure may group samples without an instruction from a user.

In this case, for example, the group generation unit 113 may perform clustering processing on analysis subject data or a result of analysis of the analysis subject data. Then the group generation unit 113 may group the samples based on a result of the clustering processing, thereby generating the first and the second sample groups.

Note that clustering processing can be performed by a known analysis method such as a group average method, a Ward's method, a nearest neighbor method, a furthest neighbor method, and a k-means method. Further, when clustering processing is performed, the group generation unit 113 may perform Uniform Manifold Approximation and Projection (UMAP) as preprocessing or analysis processing on analysis subject data.

As described above, the group generation unit 113 may group samples based on an instruction from a user, or based on analysis processing without an instruction from a user. That is, the group generation unit 113 may group samples using any method by which samples can be appropriately grouped in accordance with their features.

The text acquisition unit 114 acquires text data corresponding to each of a plurality of samples. More specifically, the text acquisition unit 114 acquires text data in which experiment contents of the samples are recorded. The text acquisition unit 114 outputs the acquired text data to the extraction unit 115.

Note that the text acquisition unit 114 may store the acquired text data in the storage device 130 each time it acquires text data. Further, the text acquisition unit 114 may read the text data from the storage device 130 and output it to the extraction unit 115 as necessary.

Further, in this case, the text acquisition unit 114 does not need to read all pieces of the text data stored in the storage device 130, and may instead read only the text data designated by a user and output it to the extraction unit 115.

Further, the text acquisition unit 114 may read the text data corresponding to the samples grouped by the group generation unit 113 and output it to the extraction unit 115.

That is, the text acquisition unit 114 may create a database in which text data is stored. Further, the text acquisition unit 114 according to this embodiment may be configured so that a user can select analysis subject data from the created database as appropriate.

Further, in the database described herein, text data may be associated with the above-described analysis subject data and stored. That is, the server 100 according to this embodiment may associate the acquired analysis subject data with text data for each sample and store them in the same database.

Note that the text data according to this embodiment is data of a document created by an experimenter who conducted an experiment on samples in order to record contents of the experiment, which data is recorded in the form of text.

For example, text data may be created by an experimenter inputting text to be recorded as experiment contents using input means such as a keyboard to the user terminal 200. In this case, the text acquisition unit 114 receives the input text data from the user terminal 200, thereby acquiring the text data in which the experiment contents of the samples are recorded. Note that, in this case, the text data may be referred to as an electronic experiment notebook.

Further, text to be recorded as experiment contents may be text recorded on a paper surface by an experimenter using a writing material etc. In this case, the text acquisition unit 114 may acquire image data of the paper surface in which the experiment contents are recorded by a user from the user terminal 200 and perform image processing on the acquired image data, thereby converting characters described in the paper surface into text data. Then the text acquisition unit 114 may acquire the converted text data as text data in which the experiment contents of the samples are recorded. Note that, in this case, text to be recorded as experiment contents may be a so-called experiment notebook.

As described above, the text acquisition unit 114 according to this embodiment may acquire text data originally recorded as electronic data, or text data generated by converting character information originally recorded on a paper surface.

That is, the text acquisition unit 114 may acquire text data by acquiring data other than text data and converting the acquired data, or by extracting it from the acquired data.

For example, the text acquisition unit 114 may extract text data from electronic data including text data and image data. Note that, an image described herein may be, for example, a photograph of the exterior of a sample, a sketch of an experimental instrument, an image showing spectrum data obtained by measuring a sample, or the like. That is, an image described herein may be any image to be described in an experiment notebook or an electronic experiment notebook.

The analysis system according to this embodiment evaluates a difference between pieces of information extracted from text data acquired by the text acquisition unit, the details of which will be described later. That is, the analysis system according to this embodiment extracts a difference between samples from a document created by an experimenter to record experiment contents, and evaluates the extracted difference.

According to the above configuration, the analysis system can extract a difference which may not be regarded as being important by a user or may not be recognized by a user. As a result, the analysis system 1 according to this embodiment can assist analysis that utilizes knowledge possessed by an experimenter.

The extraction unit 115 acquires text data from the text acquisition unit 114. The extraction unit 115 extracts extraction information about experiment contents from the text data. The extraction unit 115 outputs the extracted extraction information to the difference evaluation unit 116.

Extraction information according to this embodiment is used to assist analysis of samples. Therefore, the extraction information according to this embodiment preferably includes, for example, at least one of information about experimental conditions of samples and information about physical properties of the samples.

The extraction information extracted by the extraction unit 115 is information obtained by extracting information about experiment contents included in text data in such a manner that it can be classified or evaluated. Therefore, the extraction information according to this embodiment may include, for example, numerical data in which a plurality of variables are recorded, or identification data in which identification information about various elements related to each sample is recorded.

In the above case, the extraction unit 115 may first extract an item related to at least one of information about experimental conditions of samples and information about physical properties of the samples.

Then the extraction unit 115 may extract numerical data by converting the degree of the extracted item into numbers for each sample. Further, the extraction unit 115 may extract identification data by extracting the identification information about the extracted item from text data.

In the above case, the extraction unit 115 may, for example, refer to a database in which words related to the experimental conditions and words related to the physical properties are recorded. Then the extraction unit 115 may extract the item from text data by searching the words recorded in the database.

Further, the extraction unit 115 may extract the item, for example, by using artificial intelligence (AI) trained using text data as input data so as to output the item.

When the extraction unit 115 extracts numerical data, it may convert the degree of the item into numbers by extracting a value indicating the degree of the item described in text data.

For example, when "the reaction solution was heated at a reaction temperature of 60 degrees centigrade for one hour" is described in text data, the extraction unit 115 first extracts the "reaction temperature" and the "reaction time" as the items related to the experimental conditions. Then, for the item of the "reaction temperature", the extraction unit 115 convers the degree of the item into numbers by extracting the numerical value of "60 degrees centigrade". Further, for the item of the "reaction time", the extraction unit 115 converts the degree of the item into numbers by extracting the numerical value of "one hour".

Further, when the extraction unit 115 extracts the item related to the physical properties of samples, the extraction unit 115 may convert the degrees of the physical properties into numbers by assigning a first predetermined value to the sample that exhibits the physical property and a second predetermined value to the sample that does not exhibit the physical property.

For example, when text data in which "a product having a foaming property was obtained" is described and text data in which "a product was obtained" is described without mentioning the foaming property of the product are acquired, the extraction unit 115 first extracts the "foaming property" as the item related to the physical properties of the samples.

Then the extraction unit 115 assigns a first predetermined value (e.g., "1") to the item of the "foaming property" of the sample corresponding to the text data in which "a product having a foaming property was obtained" is described. Further, the extraction unit 115 assigns a second predetermined value (for example, "0") to the item of the "foaming property" of the sample corresponding to the text data in which "a product was obtained" is described without mentioning the foaming property of the product.

However, when the extraction unit 115 extracts the item related to the physical properties of the samples, the extraction unit 115 may simply extract identification information about whether or not the physical properties are exhibited without converting the degrees of the physical properties into numbers.

Further, the extraction unit 115 may extract a word indicating the degree of the item described in text data, and convert the degree of the item based on the extracted word into numbers.

For example, when there are text data in which "a slightly-foaming product was obtained" is described, text data in which "a foaming product was obtained" is described, and text data in which "a vigorously-foaming product was obtained" is described, the extraction unit 115 first extracts the "foaming property" as an item related to the physical properties of samples. Further, the extraction unit 115 extracts "slightly" and "vigorously" as words indicating the degree of the item.

Then the extraction unit 115 assigns a first predetermined value (e.g., "1") to the item of the "foaming property" of the sample corresponding to the text data in which "a slightly-foaming product was obtained" is described. Further, the extraction unit 115 assigns a second predetermined value (e.g., "2") larger than the first predetermined value to the item of the "foaming property" of the sample corresponding to the text data in which "a foaming product was obtained" is described. Furthermore, the extraction unit 115 assigns a third predetermined value (e.g., "3") larger than the second predetermined value to the item of the "foaming property" of the sample corresponding to the text data in which "a vigorously-foaming product was obtained" is described.

Further, the extraction unit 115 may extract identification information about each of the extracted items as extraction information.

In this case, the extraction unit 115 may, for example, extract the "production lot number of a compound" as an item and extract the corresponding "production lot number" as identification information.

Further, the extraction unit 115 may, for example, extract the "date when a sample was prepared" as an item and extract the corresponding "date" as identification information.

Further, the extraction unit 115 may extract, for example, the "sample preparation method" as an item. In this case, the extraction unit 115 may assign an identifier to the type of the "sample preparation method" in advance. Then the extraction unit 115 may identify the corresponding sample preparation method based on information recorded in text data, and record an identifier corresponding to the identified preparation method as extraction information.

As described above, the extraction unit 115 according to this embodiment extracts extraction information about experiment contents from text data. However, a method for extracting it is not limited to a particular method. That is, the extraction unit 115 according to this embodiment may extract extraction information using any method by which it is possible to extract, from text data, information with which samples can be classified and evaluated.

Note that the extraction unit 115 according to this embodiment may extract extraction information from text data by using, for example, artificial intelligence (AI).

In this case, the extraction unit 115 extracts extraction information by using artificial intelligence (AI) trained using the text data as input data so as to output extraction data in a form with which samples can be classified and evaluated in the difference evaluation unit 116 described later.

The difference evaluation unit 116 acquires identification information of samples belonging to the first and the second groups from the group generation unit 113. Then the difference evaluation unit 116 acquires extraction information corresponding to the samples belonging to the first and the second groups from the extraction unit 115.

The difference evaluation unit 116 evaluates a difference between a plurality of pieces of the extraction information. More specifically, the difference evaluation unit 116 according to this embodiment evaluates a difference between the extraction information corresponding to the first sample group and the extraction information corresponding to the second sample group. The difference evaluation unit 116 outputs a result of the evaluation to the notification unit 117.

For example, the difference evaluation unit 116 according to this embodiment may evaluate a difference between pieces of the extraction information by comparing the first sample group with the second sample group for each item extracted by the extraction unit 115. Then the difference evaluation unit 116 may output identification information of the item regarding which the difference evaluation unit 116 has evaluated that there is a difference between pieces of the extraction information to the notification unit 117.

For example, when the difference evaluation unit 116 compares the first sample group with the second sample group for an item in which numerical data is recorded, the difference evaluation unit 116 may compare the distribution ranges of numerical values or the averages of numerical values. That is, the difference evaluation unit 116 may compare the first sample group with the second sample group using any method by which numerical values or numerical distributions in the respective sample groups can be compared and then evaluated.

Then, for example, when a difference between the compared pieces of numerical data is equal to or greater than a predetermined threshold, the difference evaluation unit 116 may determine that there is a difference regarding the item, and output identification information of the item to the notification unit 117.

Further, for example, when the difference evaluation unit 116 compares the first sample group with the second sample group for an item in which identification information is recorded, the difference evaluation unit 116 may compare the first sample group with the second sample group based on identification information most frequently given to each of the groups.

Further, in this case, the difference evaluation unit 116 may compare the first sample group with the second sample group based on the percentage of the identification information given to each of the groups. Then, for example, when a difference between the percentages of the compared identification information is equal to or greater than a predetermined threshold, the difference evaluation unit 116 may determine that there is a difference regarding the item.

The notification unit 117 acquires identification information of the item regarding which it is evaluated that there is a difference from the difference evaluation unit 116. The notification unit 117 notifies a user about the item regarding which the difference evaluation unit has evaluated that there is a difference between a plurality of pieces of the extraction information.

For example, the notification unit 117 may notify a user about the item regarding which it is evaluated that there is a difference by displaying a message, for example, a message that "the experiment with the first sample group and the experiment with the second sample group were conducted on different dates", a message that "the lot number of the used material is different between the selected sample and the unselected sample", or a message that "the first sample group and the second sample group have different reaction temperature distributions" on the display apparatus of the user terminal 200.

That is, the notification unit 117 may notify a user by including the item regarding which it is evaluated that there is a difference in a message.

Further, for example, the notification unit 117 may present a list of items regarding each of which it is evaluated that there is a difference to a user.

Further, for example, the notification unit 117 may present a list of all items displayed by the extraction unit 115 to a user. Further, the notification unit 117 may highlight the item regarding which it is evaluated that there is a difference. It should be noted that the highlighting described here may include, for example, displaying the item regarding which it is evaluated that there is a difference in a color different from that of another item, and displaying a predetermined mark near the item regarding which it is evaluated that there is a difference.

According to the above configuration as well, the notification unit 117 according to this embodiment can notify a user about the item regarding which it is evaluated that there is a difference.

### (Operation of Analysis system)

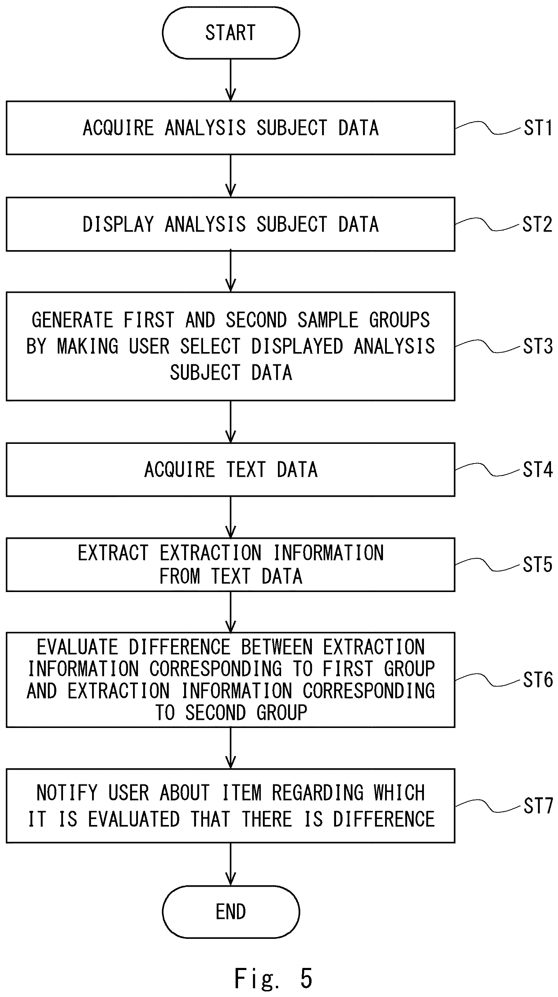

Next, operations performed by the analysis system, that is, an analysis method according to the first embodiment, will be described in detail. Fig. 5 is a flowchart showing operations performed by the analysis system according to the first embodiment. Note that, in the following description, Figs. 1 to 4 will be referred to as appropriate.

In the processing procedure shown in Fig. 5, the processor 110 of the server 100 functions as the analysis subject data acquisition unit 111, the display control unit 112, the group generation unit 113, the text acquisition unit 114, the extraction unit 115, the difference evaluation unit 116, and the notification unit 117 by loading a program(s) stored in the storage device 130 into the memory 120 and executing the loaded program(s).

In the analysis method according to this embodiment, first, the processor 110 acquires analysis subject data (Step ST1). More specifically, in Step ST1, the processor 110 acquires analysis subject data which is at least one of measurement data obtained by measuring samples and numerical data about the samples. That is, in Step ST1, the processor 110 functions as the analysis subject data acquisition unit 111.

For example, in Step ST1, the processor 110 receives selection of analysis subject data to be analyzed from the user terminal 200. Then the processor 110 acquires the selected analysis subject data from the storage device 130.

Next, the processor 110 displays pieces of analysis subject data (Step ST2). More specifically, in Step ST2, the processor 110 displays pieces of analysis subject data so that a user can select them. That is, in Step ST2, the processor 110 functions as the display control unit 112.

Next, the processor 110 generates first and second sample groups by making a user select the displayed analysis subject data (Step ST3). That is, in Step ST3, the processor 110 functions as the group generation unit 113.

Next, the processor 110 acquires text data (Step ST4). More specifically, in Step ST4, the processor 110 acquires text data about the samples. That is, in Step ST4, the processor 110 functions as the text acquisition unit 114.

Next, the processor 110 extracts extraction information from the text data (Step ST5). More specifically, in Step ST5, the processor 110 extracts extraction information about experiment contents from the text data. That is, in Step ST5, the processor 110 functions as the extraction unit 115.

Next, the processor 110 evaluates a difference between the extraction information corresponding to the first group and the extraction information corresponding to the second group (Step ST6). That is, in Step ST6, the processor 110 functions as the difference evaluation unit 116.

Lastly, the processor 110 notifies a user about the item regarding which it is evaluated that there is a difference (Step ST7), and the analysis system according to this embodiment ends the series of operations. That is, in Step ST7, the processor 110 functions as the notification unit 117.

As described above, the analysis system according to this embodiment evaluates a difference between pieces of information extracted from text data acquired by the text acquisition unit.

According to the above configuration, the analysis system can, for example, extract a difference between samples whose importance is not recognized by a user. As a result, the analysis system 1 according to this embodiment can assist analysis that utilizes knowledge possessed by an experimenter.

### <Other Embodiments>

Although the analysis system according to each of the first and the second embodiments is implemented as the server 100, the configuration of the analysis system according to the present disclosure is not limited thereto. For example, the analysis system according to the present disclosure may be implemented by two or more computer apparatuses. Further, for example, some or all of the configurations of the analysis system according to the present disclosure may be implemented as those of the user terminal 200.

Although the present disclosure has been described above with reference to the above embodiments, the present disclosure is not limited only to the configurations of the above-described embodiments. Needless to say, the present disclosure includes various modifications, changes, and combinations that can be made by a person skilled in the art within the scope of the disclosure set forth in the claims of the present application.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. An analysis system comprising:
a text acquisition unit (114) configured to acquire text data corresponding to each of a plurality of samples;
an extraction unit (115) configured to extract extraction information about the samples from the text data; and
a difference evaluation unit (116) configured to evaluate a difference between a plurality of pieces of the extraction information.

2. The analysis system according to claim 1, wherein the extraction information includes at least one of information about experimental conditions of the samples and information about physical properties of the samples.

3. The analysis system according to claim 2, wherein the extraction unit (115) extracts an item related to at least one of the information about the experimental conditions of the samples and the information about the physical properties of the samples.

4. The analysis system according to claim 3, further comprising a notification unit (117) configured to notify a user about an item regarding which the difference evaluation unit (116) has evaluated that there is a difference between the plurality of pieces of the extraction information.

5. The analysis system according to any one of claims 1 to 4, further comprising a group generation unit (113) configured to generate a first sample group and a second sample group by receiving selection of the sample from a user,
wherein the difference evaluation unit (116) evaluates a difference between the extraction information corresponding to the first sample group and the extraction information corresponding to the second sample group.

6. The analysis system according to claim 5, further comprising:
an analysis subject data acquisition unit (111) configured to acquire analysis subject data including at least one of measurement data obtained by measuring samples and numerical data about the samples; and
a display control unit (112) configured to display a plurality of pieces of the analysis subject data,
wherein the group generation unit (113) receives the selection of the sample by making a user select the displayed analysis subject data.

7. The analysis system according to any one of claims 1 to 4, further comprising:
an analysis subject data acquisition unit (111) configured to acquire analysis subject data including at least one of measurement data obtained by measuring samples and numerical data about the samples; and
a group generation unit (113) configured to perform clustering processing on at least one of the analysis subject data and a result of analysis of the analysis subject data, and generate a first sample group and a second sample group based on a result of the clustering processing,
wherein the difference evaluation unit (116) evaluates a difference between the extraction information corresponding to the first sample group and the extraction information corresponding to the second sample group.

8. An analysis method comprising:
acquiring text data corresponding to each of a plurality of samples;
extracting extraction information about the samples from the text data;
and
evaluating a difference between a plurality of pieces of the extraction information.

9. An analysis program for causing a computer to perform operations including:
acquiring text data corresponding to each of a plurality of samples;
extracting extraction information about the samples from the text data;
and
evaluating a difference between a plurality of pieces of the extraction information.
